(19) Europäisches Patentamt
European Patent Office
Office européen des brevets

(11) EP 4 083 071 A2

(12) # EUROPEAN PATENT APPLICATION

(43) Date of publication:
**02.11.2022 Bulletin 2022/44**

(21) Application number: **22155604.6**

(22) Date of filing: **14.08.2012**

(51) International Patent Classification (IPC):
***C07K 16/28*** $^{(2006.01)}$ ***A61K 39/395*** $^{(2006.01)}$

(52) Cooperative Patent Classification (CPC):
**C07K 16/2803; A61P 35/00; A61P 35/02;**
A61K 2039/505

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.08.2011 EP 11177660**
**16.08.2011 US 201161523862 P**

(62) Document number(s) of the earlier application(s) in accordance with Art. 76 EPC:
**12745883.4 / 2 744 826**

(71) Applicant: **MorphoSys AG**
**82152 Planegg (DE)**

(72) Inventors:
- **AMERSDORFFER, Jutta**
  **85421 Hebertshausen (DE)**
- **STEIDL, Stefan**
  **81241 München (DE)**
- **WINDERLICH, Mark**
  **81371 München (DE)**
- **KROHN, Susanne**
  **81241 München (DE)**
- **ROJKJAER, Lisa**
  **8908 Hedingen (CH)**

(74) Representative: **Carpmaels & Ransford LLP**
**One Southampton Row**
**London WC1B 5HA (GB)**

Remarks:
- The complete document including Reference Table(s) and the Sequence Listing(s) can be downloaded from the EPO website
- A request for correction of the description, drawings and sequence listing has been filed pursuant to Rule 139 EPC. A decision on the request will be taken during the proceedings before the Examining Division (Guidelines for Examination in the EPO, A-V, 3.).
- This application was filed on 08-02-2022 as a divisional application to the application mentioned under INID code 62.
- Claims filed after the date of filing of the application / after the date of receipt of the divisional application (Rule 68(4) EPC).

(54) **COMBINATION THERAPY WITH AN ANTI-CD19 ANTIBODY AND A PURINE ANALOG**

(57) The present disclosure describes a pharmaceutical combination of an anti-CD19 antibody and a purine analog for the treatment of non-Hodgkin's lymphoma, chronic lymphocytic leukemia and/or acute lymphoblastic leukemia.

EP 4 083 071 A2

## Description

### Cross reference

This application claims the benefit of U.S. provisional application serial number US 61/523,862 filed August 16, 2011, which is incorporated by reference in its entirety.

### Field of the Invention

The present disclosure is related to a pharmaceutical combination of an anti-CD19 antibody and a purine analog for the treatment of non-Hodgkin's lymphoma, chronic lymphocytic leukemia and/or acute lymphoblastic leukemia.

### Background

B cells are lymphocytes that play a large role in the humoral immune response. They are produced in the bone marrow of most mammals, and represent 5-15% of the circulating lymphoid pool. The principal function of B cells is to make antibodies against various antigens, and are an essential component of the adaptive immune system.

Because of their critical role in regulating the immune system, disregulation of B cells is associated with a variety of disorders, such as lymphomas, and leukemias. These include non-Hodgkin's lymphoma (NHL), chronic lymphoid leukemia (CLL) and acute lymphoblastic leukemia (ALL).

NHL is a heterogeneous malignancy originating from lymphocytes. In the United States (U.S.), the incidence is estimated at 65,000/year with mortality of approximately 20,000 (American Cancer Society, 2006; and SEER Cancer Statistics Review). The disease can occur in all ages, the usual onset begins in adults over 40 years, with the incidence increasing with age. NHL is characterized by a clonal proliferation of lymphocytes that accumulate in the lymph nodes, blood, bone marrow and spleen, although any major organ may be involved. The current classification system used by pathologists and clinicians is the World Health Organization (WHO) Classification of Tumours, which organizes NHL into precursor and mature B-cell or T-cell neoplasms. The PDQ is currently dividing NHL as indolent or aggressive for entry into clinical trials. The indolent NHL group is comprised primarily of follicular subtypes, small lymphocytic lymphoma, MALT (mucosa-associated lymphoid tissue), and marginal zone; indolent encompasses approximately 50% of newly diagnosed B-cell NHL patients. Aggressive NHL includes patients with histologic diagnoses of primarily diffuse large B cell (DLBL, DLBCL, or DLCL) (40% of all newly diagnosed patients have diffuse large cell), Burkitt's, and mantle cell. The clinical course of NHL is highly variable. A major determinant of clinical course is the histologic subtype. Most indolent types of NHL are considered to be incurable disease. Patients respond initially to either chemotherapy or antibody therapy and most will relapse. Studies to date have not demonstrated an improvement in survival with early intervention. In asymptomatic patients, it is acceptable to "watch and wait" until the patient becomes symptomatic or the disease pace appears to be accelerating. Over time, the disease may transform to a more aggressive histology. The median survival is 8 to 10 years, and indolent patients often receive 3 or more treatments during the treatment phase of their disease. Initial treatment of the symptomatic indolent NHL patient historically has been combination chemotherapy. The most commonly used agents include: cyclophosphamide, vincristine and prednisone (CVP); or cyclophosphamide, adriamycin, vincristine, prednisone (CHOP). Approximately 70% to 80% of patients will respond to their initial chemotherapy, duration of remissions last on the order of 2-3 years. Ultimately the majority of patients relapse. The discovery and clinical use of the anti-CD20 antibody, rituximab, has provided significant improvements in response and survival rate. The current standard of care for most patients is rituximab + CHOP (R-CHOP) or rituximab + CVP (R-CVP). Interferon is approved for initial treatment of NHL in combination with alkylating agents, but has limited use in the U.S. Rituximab therapy has been shown to be efficacious in several types of NHL, and is currently approved as a first line treatment for both indolent (follicular lymphoma) and aggressive NHL (diffuse large B cell lymphoma). However, there are significant limitations of anti-CD20 monoclonal antibody (mAb), including primary resistance (50% response in relapsed indolent patients), acquired resistance (50% response rate upon re-treatment), rare complete response (2% complete resonse rate in relapsed population), and a continued pattern of relapse. Finally, many B cells do not express CD20, and thus many B-cell disorders are not treatable using anti-CD20 antibody therapy.

In addition to NHL there are several types of leukemias that result from disregulation of B cells. Chronic lymphocytic leukemia (also known as "chronic lymphoid leukemia" or "CLL"), is a type of adult leukemia caused by an abnormal accumulation of B lymphocytes. In CLL, the malignant lymphocytes may look normal and mature, but they are not able to cope effectively with infection. CLL is the most common form of leukemia in adults. Men are twice as likely to develop CLL as women. However, the key risk factor is age. Over 75% of new cases are diagnosed in patients over age 50. More than 10,000 cases are diagnosed every year and the mortality is almost 5,000 a year (American Cancer Society, 2006; and SEER Cancer Statistics Review). CLL is an incurable disease but progresses slowly in most cases. Many people with CLL lead normal and active lives for many years. Because of its slow onset, early-stage CLL

is generally not treated since it is believed that early CLL intervention does not improve survival time or quality of life. Instead, the condition is monitored over time. Initial CLL treatments vary depending on the exact diagnosis and the progression of the disease. There are dozens of agents used for CLL therapy. Combination chemotherapy regimens such as FCR (fludarabine, cyclophosphamide and rituximab), and BR (bendamustine and rituximab) are effective in both newly-diagnosed and relapsed CLL. Allogeneic bone marrow (stem cell) transplantation is rarely used as a first-line treatment for CLL due to its risk.

Another type of leukemia is acute lymphoblastic leukemia (ALL), also known as acute lymphocytic leukemia. ALL is characterised by the overproduction and continuous multiplication of malignant and immature white blood cells (also known as lymphoblasts) in the bone marrow. 'Acute' refers to the undifferentiated, immature state of the circulating lymphocytes ("blasts"), and that the disease progresses rapidly with life expectancy of weeks to months if left untreated. ALL is most common in childhood with a peak incidence of 4-5 years of age. Children of age 12- 16 die more easily from it than others. Currently, at least 80% of childhood ALL are considered curable. Under 4,000 cases are diagnosed every year and the mortality is almost 1,500 a year (American Cancer Society, 2006; and SEER Cancer Statistics Review).

The human CD 19 molecule is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD 19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias (Nadler et al, J. Immunol., 131 :244-250 (1983), Loken et al, Blood, 70:1316-1324 (1987), Uckun et al, Blood, 71 :13-29 (1988), Anderson et al, 1984. Blood, 63:1424-1433 (1984), Scheuermann, Leuk. Lymphoma, 18:385-397(1995)). The expression of CD 19 on plasma cells further suggests it may be expressed on differentiated B cell tumors such as multiple myeloma, plasmacytomas, Waldenstrom's tumors (Grossbard et al., Br. J. Haematol, 102:509- 15(1998); Treon et al, Semin. Oncol, 30:248-52(2003)).

Therefore, the CD 19 antigen is a target for immunotherapy in the treatment of non-Hodgkin's lymphoma (including each the subtypes described herein), chronic lymphocytic leukemia and/or acute lymphoblastic leukemia. CD19 has also been suggested as a target for immunotherapy in the treatment of autoimmune disorders in WO2000074718, which is incorporated by reference in its entirety.

Certain CD19 therapies have been shown. T cells expressing an anti-CD19 chimeric antigen receptor (CAR) including both the CD3-ζ and CD28 molecules were administered to a patient having follicular lymphoma. Kochenderfer et al., Eradication of B lineage cell and regression of lymphoma in a patient treated with autologous T cells genetically engineered to recognize CD19, Blood, vol. 116, no: 20 (November 2010). Sadelain et al., The promise and potential pitfalls of chimeric antigen receptors, Current Opinion in Immunology, Elsevier, vol. 21, no.2, 2 April 2009, which is incorporated by reference in its entirety, also describes anti-CD19 chimeric antigen receptors (CARs). Rosenberg et al, Treatment of B cell Malignancies with T cells expressing an anti-CD19 chimeric receptor: Assessment of the Impact of Lymphocyte Depletion Prior to T cell Transfer, (September 2008), www.gemcris.od.nih.gov/ Abstracts/940_s.pdf (retrieved on 13 Jan 2012), which is incorporated by reference in its entirety, describes anti-CD19 chimeric antigen receptors (CARs) used with fludarabine. See also Eshhar et al., Proceedings of the National Academy of Sciences of USA, National Academy of Science, Washington, D.C:, vol. 90, no.2 (15 January 1993). Neither Kochenderfer et al., Sadelain et al., Rosenberg et al., nor Eshhar et al., however, describe the antibody specific for CD19 in combination with fludrabine as exemplified herein.

Fludarabine as a therapy in the treatment of CLL was described in Montserrat et al., Chronic lymphocytic leukemia treatment, Blood Review, Churchill Livingstone, vol. 7, no. 3 (1 Sept. 1993), but does not suggest the antibody specific for CD19 in combination with fludrabine as exemplified herein.

The use of a CD19 antibody in B cell disorders is discussed in US2011104150, which is incorporated by reference in its entirety, along with the cursory mention of fludarabine within a long list of potential combination partners, but fails either to teach the exemplified antibody or to suggest the synergistic effects of the combination in the treatment of non-Hodgkin's lymphoma, chronic lymphocytic leukemia and/or acute lymphoblastic leukemia as exemplified herein.

The use of a CD19 antibody in non-specific B cell lymphomas is discussed in WO2007076950, which is incorporated by reference in its entirety, along with the cursory mention of fludarabine within a long list of potential combination partners, but fails either to teach the exemplified antibody or suggest the synergistic effects of the combination in the treatment of non-Hodgkin's lymphoma, chronic lymphocytic leukemia and/or acute lymphoblastic leukemia as exemplified herein.

The use of a CD19 antibody in leukemias and lymphomas is discussed in WO2005012493, which is incorporated by reference in its entirety, along with the cursory mention of fludarabine within a long list of potential combination partners, but fails either to teach the exemplified antibody or suggest the synergistic effects of the combination in the treatment of non-Hodgkin's lymphoma, chronic lymphocytic leukemia and/or acute lymphoblastic leukemia as exemplified herein.

The use of a CD19 antibody in CLL, NHL and ALL is described in Scheuermann et al., CD19 Antigen in Leukemia

and Lymphoma Diagnosis and Immunotherapy, Leukemia and Lymphoma, Vol. 18, 385-397 (1995), which is incorporated by reference in its entirety, but fails to suggest the combination exemplified herein.

Additional antibodies specific for CD19 are described in WO2005012493 (US7109304), WO2010053716 (US12/266,999) (Immunomedics); WO2007002223 (US US8097703) (Medarex); WO2008022152 (12/377,251) and WO2008150494 (Xencor), WO2008031056 (US11/852,106) (Medimmune); WO 2007076950 (US 11/648,505 ) (Merck Patent GmbH); WO 2009/052431 (US12/253,895) (Seattle Genetics); and WO2010095031 (12/710,442) (Glenmark Pharmaceuticals), which are all incorporated by reference in their entireties.

Combinations of antibodies specific for CD19 and other agents are described in WO2010151341 (US 13/377,514) (The Feinstein Institute); US5686072 (University of Texas), and WO2002022212 (PCT/US01/29026) (IDEC Pharmaceuticals), which are all incorporated by reference in their entireties.

It is clear that in spite of the recent progress in the discovery and development of anti-cancer agents, many forms of cancer involving CD19-expressing tumors still have a poor prognosis. Thus, there is a need for improved compositions and methods for treating such forms of cancer.

## Summary

Neither alone nor in combination does the prior art suggest the synergistic effects of the combination of the exemplified antibody and fludarabine in the treatment of non-Hodgkin's lymphoma, chronic lymphocytic leukemia and/or acute lymphoblastic leukemia.

In one aspect, the present disclosure relates to a synergistic combination of an antibody specific for CD19 and a purine analog. Such combinations are useful in the treatment of B cell malignancies, such as, non-Hodgkin's lymphoma, chronic lymphocytic leukemia and/or acute lymphoblastic leukemia.

In vitro and in vivo models are considered indicative of how a certain compound or combination of compounds would behave in humans. In addition, when compounds are combined either in vitro or in vivo, one expects that the combination has only additive effects. Surprisingly, the inventors found that the combination of a particular antibody specific for CD19 and fludarabine mediated a synergistic level of specific cell killing in a chronic B-cell leukemia cell line (MEC-1) in comparison to the antibody and fludarabine alone. This *in vitro* model is indicative of how the combination will work in the treatment of chronic lymphoid leukemia (CLL) in humans. In addition, and also unexpectedly, the inventors found that the combination of a particular antibody specific for CD19 and fludarabine synergistically inhibited tumor growth and synergistically increased median survival days, both in Burkitt's lymphoma SCID mouse models, in comparison to the antibody and fludarabine alone. These *in vivo* models are indicative of how the combination will work in the treatment of non-Hodgkin's lymphoma in humans. In summary, the combination of the exemplified anti-CD19 antibody and fludarabine behaved synergistically in models relevant to NHL and CLL. As both NHL and CLL are B cell related disorders and CD19 is highly expressed on B-cells, the exemplified combination would have the same mechanism of action and should also behave synergistically in the treatment of other B cell related disorders, e.g. ALL.

Therefore, the combination of the exemplified antibody specific for CD19 and fludarabine will be effective in the treatment of humans in non-Hodgkin's lymphoma, chronic lymphocytic leukemia and/or acute lymphoblastic leukemia. In addition, the antibody specific to CD19 exemplified in the present specification has already entered into clinical trials, where such combinations can be confirmed in humans.

As the mechanism of action of fludarabine and other purine analogs are similar, as purine analogs interfere with the synthesis of nucleic acids, it is believed that synergy should also be seen when treating humans having non-Hodgkin's lymphoma, chronic lymphocytic leukemia and/or acute lymphoblastic leukemia with a combination of the exemplified anti-CD19 antibody and a purine analog other than fludarabine.

As the exemplified anti-CD19 antibody and other anti-CD19 antibodies bind CD19, it is believed that synergy should also be seen when treating humans having non-Hodgkin's lymphoma, chronic lymphocytic leukemia and/or acute lymphoblastic leukemia with a combination of any anti-CD19 antibody and a purine analog, e.g., fludarabine.

As the exemplified anti-CD19 antibody binds a specific epitope of CD19, it is believed that antibodies that cross-compete with the exemplified antibody or bind to the same epitope as the exemplified antibody should also behave synergistically when treating humans having non-Hodgkin's lymphoma, chronic lymphocytic leukemia and/or acute lymphoblastic leukemia in combination with a purine analog, e.g., fludarabine.

An aspect of the present disclosure comprises a synergistic combination wherein the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and fludarabine. In preferred aspects, the combination is used for the treatment of non-Hodgkin's lymphoma, chronic lymphocytic leukemia and/or acute lymphoblastic leukemia.

## Description of Drawings

**Figure 1** shows the cytotoxicity effects of MOR208 and fludarabine alone and in combination on MEC-1 cells.

**Figure 2** shows the ADCC dose reponse curves of the combination of MOR208 and fludarabine in MEC-1 cells.

**Figure 3** shows the amino acid sequence of the variable domains of MOR208.

**Figure 4** shows the amino acid sequence of the Fc regions of MOR208.

**Figure 5** shows the normalized specific killing of MEC-1 target cells pretreated with Fludarabine (Flu) for 72 h. The data represents a pool of 3 independent experiments with 3 different effector cell donors.

**Figure 6** shows the mean tumor growth of the MOR208, FLU, and combination (MOR208/FLU) groups of the SCID mouse model described in Example 2.

**Figure 7** shows median survival time of the MOR208, FLU, and combination (MOR208/FLU) groups of the SCID mouse model described in Example 3.

## Detailed description of the invention

"Synergy", "synergism" or "synergistic" mean more than the expected additive effect of a combination. The "synergy", "synergism" or "synergistic" effect of a combination is determined herein by the methods of Chou et al., Clarke et al., and/or Webb et al. See Ting-Chao Chou, Theoretical Basis, Experimental Design, and Computerized Simulation of Synergism and Antagonism in Drug Combination Studies, Pharmacol Rev 58:621-681 (2006), which is incorporated by reference in its entirety. See also Clarke et al., Issues in experimental design and endpoint analysis in the study of experimental cytotoxic agents in vivo in breast cancer and other models, Breast Cancer Research and Treatment 46:255-278 (1997), which is incorporated by reference in its entirety. See also Webb, J. L. (1963) Enzyme and Metabolic Inhibitors, Academic Press, New York, which is incorporated by reference in its entirety.

The term "antibody" means monoclonal antibodies, including any isotype, such as, IgG, IgM, IgA, IgD and IgE. An IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are referred to as CDR1, CDR2, and CDR3, numbered sequentially from the N-terminus. The more highly conserved portions of the variable regions outside of the CDRs are called the "framework regions". An "antibody fragment" means an Fv, scFv, dsFv, Fab, Fab' F(ab')2 fragment, or other fragment, which contains at least one variable heavy or variable light chain, each containing CDRs and framework regions.

A purine analog is an antimetabolite, which mimics the structure of metabolic purines, thereby interfering with the synthesis of nucleic acids. Fludarabine, for example, may be incorporated into RNA and DNA by substituting for the purine nucleotides, adenine and guanine. Purine analogs inhibit growth of fast proliferating cells of an individual, e.g. cancer cells, bone marrow cells or cells present in the gastrointestinal tract. Purine analogs include mercaptopurine, azathioprine, thioguanine and fludarabine.

Mercaptopurine is used in the treatment of acute leukemias, lymphomas, rheumatoid arthritis, and inflammatory bowel disease, such as Crohn's Disease and ulcerative colitis, respectively. Mercaptopurine has the following structure:

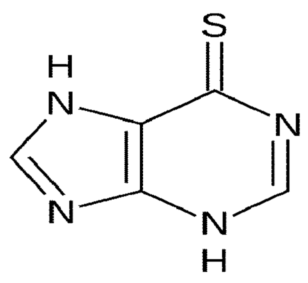

Azathioprine is the main immunosuppressive cytotoxic substance. It is widely used in transplantations to control

rejection reactions. It is nonenzymatically cleaved to 6-mercaptopurine that acts as a purine analogue and an inhibitor of DNA synthesis. By preventing the clonal expansion of lymphocytes in the induction phase of the immune response, it affects both the cell and the humoral immunity. It also successfully suppresses autoimmunity. Azathioprine has the following structure:

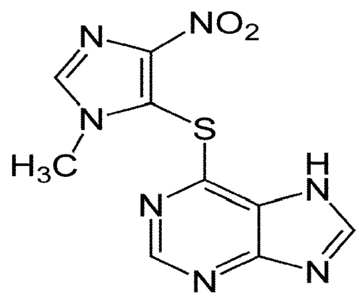

Thioguanine used during early and/or late intensification therapy of childhood acute lymphoblastic leukemia (ALL) while 6-mercaptopurine is mainly used at a different time point of therapy, namely during maintenance treatment of ALL. Thioguanine has the following structure:

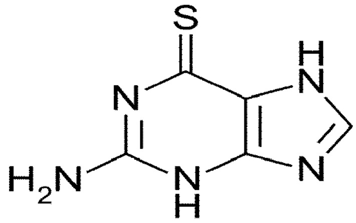

Fludarabine or fludarabine phosphate (Fludara®) is a chemotherapy drug used in the treatment of chronic lymphocytic leukemia and indolent non-Hodgkins lymphomas. Fludarabine is a purine analog. Fludarabine inhibits DNA synthesis by interfering with ribonucleotide reductase and DNA polymerase and and is S phase-specific (since these enzymes are highly active during DNA replication). Fludarabine has the following structure:

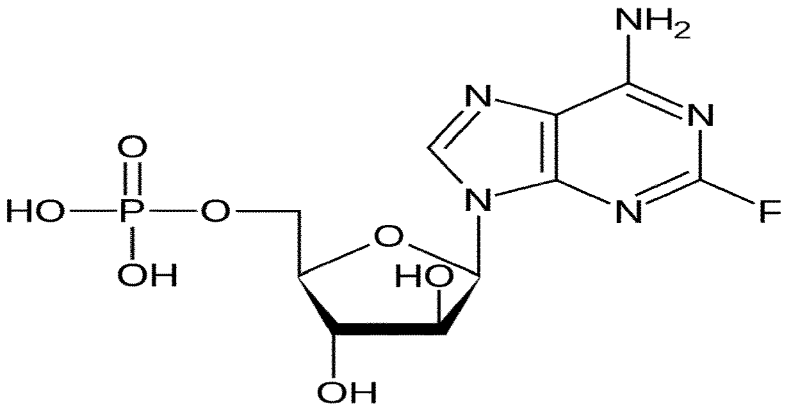

"FLU" when used herein means fludarabine.

"VH" refers to the variable region of an immunoglobulin heavy chain of an antibody, or antibody fragment. "VL" refers to the variable region of the immunoglobulin light chain of an antibody, or antibody fragment.

The term "CD19" refers to the protein known as CD19, having the following synonyms: B4, B-lymphocyte antigen CD19, B-lymphocyte surface antigen B4, CVID3, Differentiation antigen CD19, MGC12802, and T-cell surface antigen Leu-12.

Human CD19 has the amino acid sequence of:

MPPPRLLFFLLFLTPMEVRPEEPLVVKVEEGDNAVLQCLKGTSDGPTQQLTWSRESPLKPFLKLSL GLPGLGIHMRPLAIWLFIFNVSQQMGGFYLCQPGPPSEKAWQPGWTVNVEGSGELFRWNVSDLG GLGCGLKNRSSEGPSSPSGKLMSPKLYVWAKDRPEIWEGEPPCLPPRDSLNQSLSQDLTMAPGS TLWLSCGVPPDSVSRGPLSWTHVHPKGPKSLLSLELKDDRPARDMWVMETGLLLPRATAQDAGK YYCHRGNLTMSFHLEITARPVLWHWLLRTGGWKVSAVTLAYLIFCLCSLVGILHLQRALVLRRKRK RMTDPTRRFFKVTPPPGSGPQNQYGNVLSLPTPTSGLGRAQRWAAGLGGTAPSYGNPSSDVQA DGALGSRSPPGVGPEEEEGEGYEEPDSEEDSEFYENDSNLGQDQLSQDGSGYENPEDEPLGPE DEDSFSNAESYENEDEELTQPVARTMDFLSPHGSAWDPSREATSLGSQSYEDMRGILYAAPQLR SIRGQPGPNHEEDADSYENMDNPDGPDPAWGGGGRMGTWSTR. (SEQ ID NO: 7)

"MOR208" is an anti-CD19 antibody. The amino acid sequence of the variable domains is provided in Figure 3. The amino acid sequence of the heavy and light chain Fc regions of MOR208 is provided in Figure 4. "MOR208" and "XmAb 5574" are used as synonyms to describe the antibody shown in Figures 3 and 4. The MOR208 antibody is described in US patent application serial number 12/377,251, which is incorporated by reference in its entirety.

Additional antibodies specific for CD19 are described in US patent no. 7,109,304 (Immunomedics), which is incorporated by reference in its entirety; US application serial no. 11/917,750 (Medarex), which is incorporated by reference in its entirety; US application serial no. 11/852,106 (Medimmune), which is incorporated by reference in its entirety; US application serial no. 11/648,505 (Merck Patent GmbH), which is incorporated by reference in its entirety; US patent no. 7,968,687 (Seattle Genetics), which is incorporated by reference in its entirety; and US application serial no. 12/710,442 (Glenmark Pharmaceuticals), which is incorporated by reference in its entirety.

"Fc region" means the constant region of an antibody, which in humans may be of the IgG1, 2, 3, 4 subclass or others. The sequences of human Fc regions are available at IMGT, Human IGH C-REGIONs, http://www.imgt.org/IMGTrepertoire/Proteins /protein/human/IGH/IGHC/Hu_IGHCallgenes.html (retrieved on 16 May 2011).

"RefmAb33" is an antibody whose amino acid sequence is as follows:

Heavy chain including the Fc region:

QVTLRESGPALVKPTQTLTLTCTFSGFSLSTAGMSVGWIRQPPGKALEWLADIWWDDKKH YNPSLKDRLTISKDTSKNQVVLKVTNMDPADTATYYCARDMIFNFYFDVWGQGTTVTVSSASTKG PSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGGPDVFLFPPKPKDTLMIS RTPEVTCVVVDVSHEDPEVQFNWYVDGVEVHNAKTKPREEQFNSTFRVVSVLTVVHQDWLNGKE YKCKVSNKALPAPEEKTISKTKGQPREPQVYTLPPSREEMTKNQVSLTCLVKGFYPSDIAVEWESN GQPENNYKTTPPMLDSDGSFFLYSKLTVDKSRWQQGNVFSCSVMHEALHNHYTQKSLSLSPGK (SEQ ID NO: 8)

Light chain including the Fc region:

DIQMTQSPSTLSASVGDRVTITCSASSRVGYMHWYQQKPGKAPKLLIYDTSKLASGVPSRF SGSGSGTEFTLTISSLQPDDFATYYCFQGSGYPFTFGGGTKVEIKRTVAAPSVFIFPPSDEQLKSGT ASVVCLLNNFYPREAKVQWKVDNALQSGNSQESVTEQDSKDSTYSLSSTLTLSKADYEKHKVYAC EVTHQGLSSPVTKSFNRGEC (SEQ ID NO: 9)

RefmAb33 is specific for RSV, and is used as isotype control, as it shares the same Fc region as MOR208.

A "combination" means more than one item, e.g. a compound such as an antibody and fludarabine.

The present disclosure also relates to combinations, pharmaceuticals, and pharmaceutical compositions containing the described combinations. The two components of the synergistic combination of the present invention, e.g. the antibody specific for CD19 and fludarabine, may be administered together, simultaneously or separately. When administered together, the two components may be formulated together in one pharmaceutical composition, which may include a pharmaceutical acceptable carrier or excipient. Alternatively the two components might also be formulated in different pharmaceutical compositions. In this case the two components can be administered simultaneously or subsequently. In an embodiment, fludarabine, is administered prior to and/or separately from the administration of the antibody specific for CD19, e.g. MOR208.

A pharmaceutical composition includes an active agent, eg. an antibody for therapeutic use in humans. A pharmaceutical composition may include acceptable carriers or excipients.

"Administered" or "administration" includes but is not limited to delivery by an injectable form, such as, for example, an intravenous, intramuscular, intradermal or subcutaneous route or mucosal route, for example, as a nasal spray or aerosol for inhalation or as an ingestable solution, capsule or tablet.

A "therapeutically effective amount" of a compound or combination refers to an amount sufficient to cure, alleviate or partially arrest the clinical manifestations of a given disease or disorder and its complications. The amount that is effective for a particular therapeutic purpose will depend on the severity of the disease or injury as well as on the weight and general state of the subject. It will be understood that determination of an appropriate dosage may be achieved, using routine experimentation, by constructing a matrix of values and testing different points in the matrix, all of which is within the ordinary skills of a trained physician or clinical scientist.

The "CDRs" herein are defined by either Chothia et al or Kabat et al. See Chothia C, Lesk AM. (1987) Canonical structures for the hypervariable regions of immunoglobulins. J Mol Biol., 196(4):901-17, which is incorporated by reference in its entirety. See Kabat E.A, Wu T.T., Perry H.M., Gottesman K.S. and Foeller C. (1991). Sequences of Proteins of Immunological Interest. 5th edit., NIH Publication no. 91-3242, US Dept. of Health and Human Services, Washington, DC, which is incorporated by reference in its entirety.

"Cross competes" means the ability of an antibody or other binding agent to interfere with the binding of other antibodies or binding agents to CD19 in a standard competitive binding assay. The ability or extent to which an antibody or other binding agent is able to interfere with the binding of another antibody or binding molecule to CD19, and, therefore whether it can be said to cross-compete according to the invention, can be determined using standard competition binding assays. One suitable assay involves the use of the Biacore technology (e.g. by using the BIAcore 3000 instrument (Biacore, Uppsala, Sweden)), which can measure the extent of interactions using surface plasmon resonance technology. Another assay for measuring cross-competing uses an ELISA-based approach. A high throughput process for "epitope binning" antibodies based upon their cross-competition is described in International Patent Application No. WO 2003/48731

The term "epitope" includes any protein determinant capable of specific binding to an antibody or otherwise interacting with a molecule. Epitopic determinants generally consist of chemically active surface groupings of molecules such as amino acids or carbohydrate or sugar side chains and can have specific three-dimensional structural characteristics, as well as specific charge characteristics. An epitope may be "linear" or "conformational." The term "linear epitope" refers to an epitope with all of the points of interaction between the protein and the interacting molecule (such as an antibody) occur linearally along the primary amino acid sequence of the protein (continuous). The term "conformational epitope" refers to an epitope in which discontinuous amino acids that come together in three dimensional conformation. In a conformational epitope, the points of interaction occur across amino acid residues on the protein that are separated from one another.

"Binds the same epitope as" means the ability of an antibody or other binding agent to bind to CD19 and having the same epitope as the exemplified antibody. The epitopes of the exemplified antibody and other antibodies to CD19 can be determined using standard epitope mapping techniques. Epitope mapping techniques, well known in the art. include Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871 ; Geysen et al, (1984) Proc. Natl. Acad. Sci. USA 8:3998-4002; Geysen et al, (1985) Proc. Natl. Acad. Sci. USA 82:78-182; Geysen et al, (1986) Mol. Immunol. 23 :709-715. Similarly, conformational epitopes are readily identified by determining spatial conformation of amino acids such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al, (1981) Proc. Natl. Acad. Sci USA 78:3824-3828; for determining antigenicity profiles, and the Kyte-Doolittle technique, Kyte et al, (1982) J.Mol. Biol. 157: 105-132; for hydropathy plots.

**Embodiments**

An aspect of the present disclosure comprises a combination of an antibody specific for CD19 and a purine analog for use in the treatment of non-Hodgkin's lymphoma, chronic lymphocytic leukemia and/or acute lymphoblastic leukemia. In embodiments, the combination is synergistic.

Herein, the combination of the exemplified anti-CD19 antibody and fludarabine behaved synergistically in *in vitro* and *in vivo* models relevant to NHL and CLL. As both NHL and CLL are B cell related disorders and CD19 is highly expressed on B-cells, the exemplified combination should have the same mechanism of action and should also behave synergistically in the treatment of other B cell related disorders, e.g. ALL. Therefore, the combination of the exemplified antibody specific for CD19 and fludarabine will be effective in the treatment of humans in non-Hodgkin's lymphoma,

chronic lymphocytic leukemia and/or acute lymphoblastic leukemia.

As the mechanism of action of fludarabine and other purine analogs are similar, in that purine analogs interfere with the synthesis of nucleic acids, it is believed that synergy should also be seen when treating humans having non-Hodgkin's lymphoma, chronic lymphocytic leukemia and/or acute lymphoblastic leukemia with a combination of the exemplified anti-CD19 antibody and a purine analog other than fludarabine, e.g., mercaptopurine, azathioprine, and thioguanine.

As the exemplified anti-CD19 antibody and other anti-CD19 antibodies bind to the CD19 antigen, it is believed that synergy should also be seen when treating humans having non-Hodgkin's lymphoma, chronic lymphocytic leukemia and/or acute lymphoblastic leukemia with a combination of any anti-CD19 antibody and a purine analog, where the anti-CD19 antibody is, for example, described in US patent application serial number 12/377,251 (Xencor), WO2005012493, WO2010053716 (Immunomedics); WO2007002223 (Medarex); WO2008022152 (Xencor); WO2008031056 (Medimmune); WO 2007/076950 (Merck Patent GmbH); WO 2009/052431 (Seattle Genetics); and WO2010095031 (Glenmark Pharmaceuticals), all of which are incorporated by reference in their entireties.

In embodiments, the antibody specific for CD19 comprises an antibody that cross-competes with the antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises an antibody that binds to the same epitope as an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6)..

In embodiments, the antibody specific for CD19 comprises a variable heavy chain of the sequence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFDYWG QGTLVTVSS (SEQ ID NO: 10) and a variable light chain of the sequence DIVMTQSPATLSLSPGERATLSCRSSKSLQNVNGNTYLYWFQQKPGQSPQLLIYR MSNLNSGVPDRFSGSGSGTEFTLTISSLEPEDFAVYYCMQHLEYPITFGAGTKLEIK (SEQ ID NO: 11).

In embodiments, the antibody specific for CD19 comprises a heavy chain constant domain of the sequence

ASTKGPSVFPLAPSSKSTSGGTAALGCLVKD YFPEPVTVSWNSGALTSGVH
TFPAVLQSSGLYSLSSWTVPSSSLGTQTYICNVNHKPSNTKVDKKVEPKSCDKTHTCPPCPAPELL
GGPDVFLFPPKPKDTLMISRTPEVTCVVVDVSHEDPEVQFNWYVDGVEVHNAKTKPREEQFNSTF
RVVSVLTVVHQDWLNGKEYKCKVSNKALPAPEEKTISKTKGQPREPQVYTLPPSREEMTKNQVSL
TCLVKGFYPSDIAVEWESNGQPENNYKTTPPMLDSDGSFFLYSKLTVDKSRWQQGNVFSCSVMH
EALHNHYTQKSLSLSPGK. (SEQ ID NO: 12)

In embodiments, the antibody specific for CD19 comprises a light chain constant domain of the sequence

RTVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC. (SEQ ID NO: 13)

In embodiments, the purine analog is fludarabine.

In embodiments, the components of the combination, the antibody specific for CD19 and fludarabine, are administered separately. In an embodiment, fludarabine is administered prior to administration of the antibody specific for CD19.

In embodiments the combination is a pharmaceutical composition. In embodiments, the composition comprises

an acceptable carrier. In embodiments, the combination is administered in an effective amount.

In an aspect the synergistic combination of an antibody specific for CD19 comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and fludarabine is able to mediate killing of MEC-1 cells by ADCC in the presence of isolated human PBMCs with an at least two-fold, three-fold, four-fold, or five-fold better efficacy than fludarabine alone.

An aspect of the present disclosure comprises a synergistic combination of an antibody specific for CD19 comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and fludarabine for the treatment of non-Hodgkin's lymphoma, chronic lymphocytic leukemia and/or acute lymphoblastic leukemia. In embodiments, the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

Another aspect comprises a method of treating non-Hodgkin's lymphoma, chronic lymphocytic leukemia and/or acute lymphoblastic leukemia in an individual in need thereof, which method comprises administration of an antibody specific for CD19 and a purine analog. In embodiments of the method, the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6). In embodiments of the method, the purine analog is fludarabine.

## Examples

Example 1: Inhibition of proliferation of MEC-1 cells using MOR208 and fludarabine alone and in combination

### Materials

MEC-1 cells: chronic B-cell leukemia cell line DSMZ# ACC497; Cell Medium: Iscove's Modified Dulbecco's Medium (IMDM) with GlutaMAX™, Invitrogen, Cat No.: 31980-048, 20% FCS; PBMCs: RPMI1640, with stabile Glutamine, PAN Biotech GmbH, Cat No.: P04-13500 supplemented with 10% FCS; Biocoll: Biochrome AG CAT No.: L6115 LOT No.: 1050T; Fludarabine: Bayer, 25 mg/ml in ddH2O, LOT No.: 9100ST; and RefmAb33 (anti-RSV) with same Fc region as MOR208.

### Methods

The cytotoxicity of MOR208 and fludarabine alone and in combination was tested in MEC-1 cells. FLU is a purine analog, therefore, functions via direct cytoxicity in MEC-1 cells. MOR208 targets CD19 and additionally functions via ADCC in killing MEC-1 cells. For the following groups MEC-1 cell killing was measured: FLU at 18μg/ml; MOR208 at 66pm and the combination of MOR208 at 66pm and FLU at 18μg/ml. These concentrations were chosen as they are near or at the EC50 for MOR208 and FLU.

In the FLU group and MOR208+FLU combination group, the MEC-1 cells were pre-incubated with FLU for 72 hours prior to the ADCC assay measurements. The MEC-1 cells were stained using 1mg/ml Calcein AM then counted and adjusted to $2\times10^5$/ml. The PBMCs were counted and adjusted to $6\times10^6$/ml. The ADCC assays were done as follows: Using 96 well plates, a 100μl cell suspension of MEC-1 cells was added per well, then 100μl cell suspension of PBMCs was added to each well resulting in an E:T ratio of 30:1. The antibodies were diluted to 1μg/ml in medium. Cells were centrifuged and re-suspended. To the target:effector cell-pellet 100μl antibody solution or according control solution was added. The mixture was incubated for 4h in CO2-incubator at 37°C. The ADCC measurements were taken as follows: the incubated cell solution (~100μl) was transfered into FACS tubes and 200μl FACS buffer (DPBS + 3%FCS) and 0,5 μl PI stock solution was added to each tube. FACS-Calibur was used. Dead MEC-1 cells were stained with propidium iodide.

Table 1 and Figure 1 show the raw data.

Table 1

| | control | MOR208 66pm | FLU 18μg/ml | FLU+MOR208 combination |
|---|---|---|---|---|
| Experiment 1 | 11 | 35,2 | 36,39 | 52,14 |

(continued)

| | control | MOR208 66pm | FLU 18μg/ml | FLU+MOR208 combination |
|---|---|---|---|---|
| Experiment 2 | 19,5 | 29,8 | 38,48 | 46,9 |
| Experiment 3 | 29,9 | 47,01 | 57,27 | 65,63 |

The values represent % dead cells. Each experiment represents PBMCs from different donors. The contol used for Experiments 1 and 2 was RefMab33. The control used for Experiment 3, was PBMCs alone.

Table 2 shows the raw data of Table 1 normalized for specific killing and the results of the Chou calculations done in the determination of synergism.

Table 2

| | Fludarabine 18 μg/ml | MOR20866 pM | Flu + MOR208 (combination) | Chou Index |
|---|---|---|---|---|
| Experiment 1 | 0,6 | 0,6 | 1,0 | 0,03 |
| Experiment 2 | 0,7 | 0,4 | 1,0 | 0,3 |
| Experiment 3 | 0,8 | 0,5 | 1,0 | 0,3 |
| Average | 0,7 | 0,5 | 1,0 | 0,2 |

The values shown in Table 2 are calculated as follows: 1) from the raw data (% dead cells) shown in Table 1, the background (controls) were subtracted, resulting in the specific killing for each treatment group; then 2) the specific killing values were normalized by setting the combination of MOR208 + FLU to 1. The averages of Table 2 are depicted in Figure 5. Example ADCC dose response curves used in the Chou factor calculations of the MOR208 + FLU combination are shown in Figure 2.

Chou Index (CI) calculations were completed in order to determine synergy of the combination of the exemplified anti-CD19 antibody and fludarabine as compared to MOR208 and FLU alone. The calculations are described in Ting-Chao Chou, Theoretical Basis, Experimental Design, and Computerized Simulation of Synergism and Antagonism in Drug Combination Studies, Pharmacol Rev 58:621-681 (2006), which is incorporated by reference in its entirety and Chou TC, Talalay P, Quantitative analysis of dose-effect relationships: the combined effects of multiple drugs or enzyme inhibitors. Adv Enzyme Regul 22: 27-55 (1984), which is incorporated by reference in its entirety. The methods of Chou-Talalay are carried out using the CI-isobol method.

Median-effect equation

The median-effect equation models of the effect of an inhibitor (such as a drug) as $F_a/F_u$ =(D/D50)^m, where D is the dose, $F_a$ and $F_u$ is the fraction of the system affected and unaffected by the dose D ($F_a + F_u = 1$); D50 is the dose producing the median effect (e.g. IC50, ED50, LD50). The constant m determines the shape of the dose-effect curve. We used Excel Fit software to carry out a linear regression calculation to estimate the parameters m and D50.

The effects of the combination on MEC-1 cells is measured % cell death as described above. We define the fraction $F_u$ to be the ratio of % cell death of the treated cell line to the % cell death of the cell line exposed to a control. That is:

$$F_u = \text{\% cell death(treated cell line)/ \% cell death (non-treated cell line)}$$

Then the % cell death of a cell line is the constant D50 in the median effect equation, which can be estimated by the linear regression described above.

CI-isobol method

The CI-isobol method provides a quantitative assessment of synergism between drugs. A combination index (CI) is estimated from dose-effect data of single and combined drug treatments. A value of CI less than 1 indicates synergism; CI = 1 indicates additive effect; and CI > 1 indicates antagonism. Drug interaction (synergism or antagonism) is more pronounced the farther a CI value is from 1.

Formally, the combination index (CI) of a combined drug treatment is defined as $CI = D_1/D_{x1} + D_2/D_{X2}$. Here D1 and D2 are the doses of drug 1 and drug 2 of the combination, respectively; and Dx1, and Dx2 is the dose of a

treatment with only drug 1 and drug 2 that would give the same effect as that of the combination. The doses Dx1 and Dx2 need to be estimated from the dose-effect data of single drug treatments. Essentially, a median effect equation is fitted to the data of each drug. From the median effect equation of a drug, we can estimate the dose (i.e. D) necessary to produce an effect (i.e. Fa, Fu). The further a point lies from the additive line, the bigger the different between 1 and its CI, thus the stronger the (synergistic or antagonistic) effect is.

Results

As shown in Table 2, the Chou index values indicate clear synergism of the combination of MOR208 and fludarabine in the specific killing of MEC-1 cells as compared to MOR208 and fludarabine alone. This conclusion is based upon the Chou calculations of 0.03, 0.3 and 0.3 in each of the three experiments, respectively, with an average of 0.21, where a CI <1 indicates synergism. Therefore, the combination of MOR208 and fludarabine will also behave synergistically in the treatment of non-Hodgkin's lymphoma (NHL), chronic lymphoid leukemia (CLL), and/or acute lymphoblastic leukemia (ALL) in humans.

In order to confirm the results of the above Chou calculations, the normalized data of Table 2 was evaluated for statistical significance using the Bonferroni's Multiple Comparison Test. See James, et al, Antibody-mediated B-cell depletion before adoptive immunotherapy with T cells expressing CD20-specific chimeric T-cell receptors facilitates eradication of leukemia in immunocompetent mice, Blood, 114(27):5454-63 (Epub 2009 Oct 30), which is incorporated by reference in its entirety. The results are shown in Table 3.

Table 3

| Bonferroni's Multiple Comparison Test | Mean Diff. | T value | Significant? (P < 0.05) | Summary |
|---|---|---|---|---|
| Fludarabine (18μg/ml) vs. FLU + MOR 208 combination | -0.3067 | 5.039 | Yes | ** |
| MOR208 (66pM) vs. FLU + MOR208 combination | -0.5167 | 8.490 | Yes | *** |
| ** p < 0,01<br>*** p < 0,001 | | | | |

Results

As shown in Table 3, the Bonferroni's Multiple Comparison Test shows that the combination treatment of FLU + MOR208 is statistically more effective in the specific killing of MEC-1 cells than the treatment of FLU and MOR208 alone.

Example 2: MOR208 and FLU alone and in combination in subcutaneously (SC)-implanted human Ramos Burkitt's B-cell lymphoma tumor growth model.

Materials

RAMOS human Burkitt's lymphoma cells (ATCC number CRL-1596, lot# 3953138); Vehicle control: 0.9% sodium chloride, 25mg/ml mannitol, pH 7.0;; SCID Mice (University of Adelaide, Waite Campus, Urrbaraie, SA, Australia, Strain C.B.-17-Igh-$1^{b}$-$Prkdc^{scid}$).

Methods

Six-to-seven-week old female C.B-17 SCID mice were implanted sub-cutaneously with RAMOS cells (~5 $\times$ $10^6$ cells/mouse). 14 days after inoculation, the mice were separated into ten groups of eight, where each group had tumor volumes of relatively the same size. Treatment began on Day 14. The treatment regimens are provided in Table 4. The study duration was 63 days.

Table 4

| No. of Animals | Test Articles | Dose (mg/kg) | Treatment Route and Schedule |
|---|---|---|---|
| 8 | Fludarabine | 125 | IP, Q1Dx5 |
| 8 | Fludarabine | 250 | IP, Q1Dx5 |

(continued)

| No. of Animals | Test Articles | Dose (mg/kg) | Treatment Route and Schedule |
|---|---|---|---|
| 8 | MOR208 | 1*->10 | Dys 14*, 17*, 21*, 24, 28, 31, 35, and 38 |
| 8 | Vehicle | | IP, Q1Dx5 |
| 8 | MOR208/ Fludarabine | 1*-> 10/ 125 | Dys 14*, 17*, 21*, 24, 28, 31, 35, and 38/ IP, Q1Dx5 |

MOR208, fludarabine, and the vehicle were administered in a volume of 0.1 mL/10 g of body weight. The initial readout was tumor growth, specifically tumor volume at study day 38. Tumor weights were calculated using the equation (I × w2)/2, where I and w refer to the larger and smaller dimensions collected at each measurement.

The results are shown in Table 5 and the averages are depicted in Figure 6.

Table 5

| Tumor Volume [mm^3] at study day 38 | | | | | |
|---|---|---|---|---|---|
| | Vehicle Control | MOR208 10 mg/kg | Fludarabine 125 mg/kg | Fludarabine 250 mg/kg | Combination: MOR208/FLU 125 |
| Group 1 | 2890 | 2138 | 1666 | 1352 | 1268 |
| Group 2 | 4400 | 2025 | 2560 | 1352 | 750 |
| Group 3 | 4200 | 3179 | 864 | 2816 | 726 |
| Group 4 | 4152 | 1764 | 1913 | 1764 | 446 |
| Group 5 | 3791 | 1862 | 3564 | 650 | 936 |
| Group 6 | 4513 | 3402 | 2560 | 787 | 1268 |
| Group 7 | 4152 | 2560 | 2025 | 1800 | 787 |
| Group 8 | | 2816 | 1437 | 787 | |
| **Average** | **4014** | **2468** | **2073** | **1413** | **883** |

In addition to an endpoint of tumor volume at study day 38, the parameters of 1) reduced tumor growth [%] at day 38, and 2) increased time to 4000mg tumors [%] were evaluated. The results are shown in Table 6.

Table 6

| Treatment Groups | Reduced tumor growth at study Day 38 (%) | Increased time to 4000mg [%] |
|---|---|---|
| MOR208 | 38.5 | 17.52 |
| FLU 125 | 48.3 | 22.37 |
| FLU 250 | 64.8 | 30.19 |
| Combination of MOR208 and FLU 125 | 78 | 59.3 |
| Control | 0 | 0 |

Both of the endpoints shown in Table 6 were evaluated using the Fractional Product Concept (FPC) in order to determine if synergism existed with the treatment of the combination of MOR208 and FLU treatment as compared to MOR208 and Fludarabine alone. When the combination treatment group is more effective than the FPC calculation, then synergism exists. The Fractional Product Concept was calculated using the formula 1-[(1-A)*(1-B)] = fpc(%), as described by Webb, J. L. (1963) Enzyme and Metabolic Inhibitors, Academic Press, New York, which is incorporated by reference in its entirety. After the FPC calculations were completed, the resulting FPC value and the values from Table 6 were normalized by setting the FPC value to 1. The results of these calculations are shown in Table 7.

Table 7

| | MOR208 [10 mg/kg] | Fludarabine [125 mg/kg] | Fludarabine [250 mg/kg] | Fractional Product combination | MOR208 + FLU 125 combination | Effect |
|---|---|---|---|---|---|---|
| Reduced tumor growth at study Day 38 (%) | 0.57 | 0.75 | 0,9 | 1 | 1.15 | Synergism |
| Increased time to 4000mg [%] | 0.48 | 0.62 | 0,8 | 1 | 1.65 | Synergism |

Results

Both of the endpoints are a measure of inhibition of tumor growth and in both endpoints, reduced tumor growth (%) at study day 38 and increased time (%) to 4000mg, the combination of MOR208 + FLU125 showed clear synergism in comparision to MOR208 and FLU alone.

In order to confirm the results of the above FPC calculations, the data of average Tumor Volume [mm^3] at study day 38 (shown in Table 5) was evaluated for statistical significance using the Bonferroni's Multiple Comparison Test. The results are shown in Table 8.

Table 8

| Treatment Group | Bonferroni's Multiple Comparison Test; p value |
|---|---|
| FLU125 vs FLU125 + MOR208 (combination) | $p < 0.01$ |
| FLU250 vs FLU125 + MOR208 (combination) | n.s. |
| MOR208 vs FLU125 + MOR208 (combination) | $p < 0.001$ |
| Control vs. FLU 125 | $p < 0.001$ |
| Control vs. FLU250 | $p < 0.001$ |
| Control vs. FLU125 + MOR208 (combination) | $p < 0.001$ |
| Control vs. MOR208 | $p < 0.001$ |

A p value of < 0.05 shows statistical significance.

Results

As shown in Tables 5 and 6, in all parameters, the combination of MOR208 and Fludarabine 125 mg/kg was more effective in the inhibition of tumor growth *in vivo* than MOR208 or Fludarabine alone. This increase in effectiveness of the combination of MOR208 and Fludarabine 125 mg/kg in the inhibition of tumor growth *in vivo* as compared to both MOR208 and Fludarabine alone was confirmed to be statistically significant, as shown in Table 8. In addition, the Fractional Product Concept calculations show clear synergism of combination of MOR208 and Fludarabine 125 mg/kg in the inhibition of tumor growth *in vivo* as compared to MOR208 or Fludarabine alone, as shown in Table 7. Therefore, the combination of MOR208 and fludarabine will also behave synergistically in the treatment of non-Hodgkin's lymphoma (NHL), chronic lymphoid leukemia (CLL), and acute lymphoblastic leukemia (ALL) in humans.

In addition, the combination of MOR208 and Fludarabine 125 mg/kg was more effective in the inhibition of tumor growth *in vivo* than the higher dose Fludarabine 250 mg/kg alone. As fludarabine has shown significant side effects at higher doses, this result shows that a safer, more effective alternative to high dose of Fludarabine, is the combination of MOR208 and Fludarabine.

Example 3 MOR208 and fludarabine alone and in combination in human Non-hodgkin RAMOS tumor in SCID mice, survival model

Materials

Cyclophosphamide (Fluka, Buchs Switzerland, Lot. No. 07551661); Vehicle Control: 0.9% sodium chloride, 25mg/ml mannitol, pH 7.0; SCID Mice (University of Adelaide, Waite Campus, Urrbaraie, SA, Australia, Strain C.B.-17-Igh-$1^b$-$Prkdc^{scid}$); RAMOS human lymphoma cells (ATCC number CRL-2638).

Methods

SCID mice were pre-treated with Cyclophosphamide (18 mg/kg, i.p., twice daily) for two days prior to RAMOS cell inoculation (Day -5 and -4). On the day of inoculation (Day -3), the mice were separated into ten groups of eight mice each, and inoculated with $1 \times 10^6$ RAMOS cells each intravenously into the tail vein. The dosing regimen for each group is shown in Table 9 and commenced on Day 0. The study duration was three weeks.

Table 9: Dosing regimen

| Group | Compound | Treatment | Intended Schedule |
|---|---|---|---|
| A | Fludarabine | 125 mg/kg, i.p, in 10 mL/kg | Once daily (Day 0-5) |
| B | MOR208 | 3 mg/kg, i.v., in 10 mL/kg | Twice weekly for 3 weeks |
| C | Vehicle Control | i.p., 10 mL/kg | Once daily (Day 0-5) |
| AB | Fludarabine /MOR208 | 125 mg/kg, i.p; 3 mg/kg, i.v. in 10 mL/kg; | Once daily (Day 0-5) /twice weekly for 3 weeks |
| E | Fludarabine | 250 mg/kg, i.p, in 10 mL/kg | Once daily (Day 0-5) |

The readout was median survival time in days. In order to evaluate whether the effect of the combination as compared to the individual treatment groups was synergistic, the methods of Clark et al. were used.

Clarke et al. synergism

The method is described in Issues in experimental design and endpoint analysis in the study of experimental cytotoxic agents *in vivo* in breast cancer and other models, Breast Cancer Research and Treatment 46:255-278 (1997), which is incorporated by reference in its entirety.

The data is analysed in the following way:

| | |
|---|---|
| Antagonistic | $(AB)/C < (A/C) \times (B/C)$ |
| Additive | $(AB)/C = (A/C) \times (B/C)$ |
| Synergistic | $(AB)/C > (A/C) \times (B/C)$ |

where A is the treatment with MOR208; B is the treatment with FLU alone; C is the response to the treatment vehicle; AB is the combination of treatments A and B. The median survival time in days for each study group and the Clarke et al. analysis are shown in Table 10.

Table 10

| | Median Survival Time (Days) |
|---|---|
| A = response to treatment MOR208 | 23,5 |
| B = response to treatment FLU | 22 |
| C = response to treatment vehicle | 18,5 |
| AB = combination of treatments A and B | 31 |
| | |
| (AB)/C | 1,675675676 |
| | is bigger than |

(continued)

| | Median Survival Time (Days) |
|---|---|
| (A/C) × (B/C) | 1,510591673 |
| | = Synergism |

Results

The combination of MOR208 and FLU showed clear synergism in median survival days as compared to MOR208 and FLU alone.

In order to confirm the results of the above synergism calculations, the median survival time in days of the combination of MOR208 and fluradabine 125 mg/kg was compared to MOR208 and fluradabine alone using the Mantel-Haenszel test [P value], and Gehan-Wilcoxon test [P value]. Results shown in Table 11.

Table 11

| Treatment Group | Mantel-Haenszel test [P value] | Gehan-Wilcoxon test [P value] |
|---|---|---|
| FLU/MOR vs. FLU 125 | 0,0008 | 0,0012 |
| FLU/MOR vs. MOR | 0,0001 | 0,0004 |
| FLU/MOR vs. FLU250 | 0,0011 | 0,0016 |
| FLU/MOR vs. control | 0,0001 | 0,0004 |
| Control vs. FLU 125 | 0,0061 | 0,0162 |
| Control vs. FLU250 | 0,0061 | 0,0162 |
| Control vs. MOR208 | < 0.0001 | 0,0002 |

Results

Table 10 shows that the combination of MOR208 and Fludarabine 125 mg/kg synergistically increased the median survival time in the Burkitt's lymphoma SCID mouse model as compared to both MOR208 and Fludarabine alone. This increase of median survival time of the combination of MOR208 and Fludarabine 125 mg/kg *in vivo* was confirmed to be statistically significant as compared to both MOR208 and Fludarabine alone, as shown in Table 11. Therefore, the combination of MOR208 and fludarabine will also behave synergistically in the treatment of non-Hodgkin's lymphoma (NHL), chronic lymphoid leukemia (CLL), and acute lymphoblastic leukemia (ALL) in humans.

In addition, the combination of MOR208 and Fludarabine 125 mg/kg was more effective in increasing median survival time *in vivo* than the higher dose Fludarabine 250 mg/kg alone. As fludarabine has shown significant side effects at higher doses, despite its effectiveness, this result shows that a safer, more effective alternative to high dose Fludarabine, is the combination of MOR208 and Fludarabine.

It is to be understood that the description, specific examples and data, while indicating exemplary embodiments, are given by way of illustration and are not intended to limit the present invention. Various changes and modifications within the present invention will become apparent to the skilled artisan from the discussion, disclosure and data contained herein, and thus are considered part of the invention.

The invention further provides the following non-limiting embodiments:

1. A synergistic combination of an antibody specific for CD19 comprising an antibody that cross-competes with an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and fludarabine for use in the treatment of non-Hodgkin's lymphoma, chronic lymphocytic leukemia and/or acute lymphoblastic leukemia.

2. A combination according to embodiment 1, wherein the antibody comprises an antibody that binds to the same epitope as an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1

region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

3. A combination according to embodiment 1 or 2, wherein the antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

4. A combination according to any one of the preceding embodiments, wherein the antibody comprises a variable heavy chain of the sequence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFDYWG QGTLVTVSS (SEQ ID NO: 10) and a variable light chain of the sequence DIVMTQSPATLSLSPGERATLSCRSSKSLQNVNGNTYLYWFQQKPGQSPQLLIYR MSNLNSGVPDRFSGSGSGTEFTLTISSLEPEDFAVYYCMQHLEYPITFGAGTKLEIK (SEQ ID NO: 11).

5. A combination according any one of the preceding embodiments, wherein the antibody comprises a heavy chain constant domain of the sequence

ASTKGPSVFPLAPSSKSTSGGTAALGCLVKD YFPEPVTVSWNSGALTSGVH
TFPAVLQSSGLYSLSSWTVPSSSLGTQTYICNVNHKPSNTKVDKKVEPKSCDKTHTCPPCPAPELL
GGPDVFLFPPKPKDTLMISRTPEVTCVVVDVSHEDPEVQFNWYVDGVEVHNAKTKPREEQFNSTF
RVVSVLTVVHQDWLNGKEYKCKVSNKALPAPEEKTISKTKGQPREPQVYTLPPSREEMTKNQVSL
TCLVKGFYPSDIAVEWESNGQPENNYKTTPPMLDSDGSFFLYSKLTVDKSRWQQGNVFSCSVMH
EALHNHYTQKSLSLSPGK(SEQ ID NO: 12).

6. A combination according to any one of the preceding embodiments, wherein said antibody specific for CD19 and fludarabine are administered separately.

7. A combination according to any one of the preceding embodiments, wherein fludarabine is administered prior to administration of the antibody specific for CD19.

8. A combination according to any one of the preceding embodiments, which is able to mediate killing of MEC-1 cells by ADCC in the presence of isolated human PBMCs with an at least two-fold better efficacy than fludarabine alone.

9. A combination according to any one of the preceding embodiments for use in the treatment of non-Hodgkin's lymphoma, wherein the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

## Claims

**1.** A combination of an antibody specific for CD19 and a purine analog for use in the treatment of non-Hodgkin's lymphoma, chronic lymphocytic leukemia or acute lymphoblastic leukemia, wherein the antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6), and wherein the purine analog is mercaptopurine, azathioprine or thioguanine.

**2.** An antibody specific for CD19 wherein said antibody comprises an HCDR1 region comprising sequence SYVMH (SEQ ID NO: 1), an HCDR2 region comprising sequence NPYNDG (SEQ ID NO: 2), an HCDR3 region comprising

sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6) for use in combination with a purine analog in the treatment of non-Hodgkin's lymphoma, chronic lymphocytic leukemia and/or acute lymphoblastic leukemia, wherein the purine analog is mercaptopurine, azathioprine or thioguanine.

**3.** A purine analog for use in combination with an antibody specific for CD19 wherein said antibody comprises an HCDR1 region comprising sequence SYVMH (SEQ ID NO: 1), an HCDR2 region comprising sequence NPYNDG (SEQ ID NO: 2), an HCDR3 region comprising sequence GTYYYGTRVFDY (SEQ ID NO: 3), an LCDR1 region comprising sequence RSSKSLQNVNGNTYLY (SEQ ID NO: 4), an LCDR2 region comprising sequence RMSNLNS (SEQ ID NO: 5), and an LCDR3 region comprising sequence MQHLEYPIT (SEQ ID NO: 6) in the treatment of non-Hodgkin's lymphoma, chronic lymphocytic leukemia and/or acute lymphoblastic leukemia, and wherein the purine analog is mercaptopurine, azathioprine or thioguanine.

**4.** The combination for use according to any one of the preceding claims, wherein the antibody comprises a variable heavy chain of the sequence EVQLVESGGGLVKPGGSLKLSCAASGYTFTSYVMHWVRQAPGKGLEWIGYINPYNDG TKYNEKFQGRVTISSDKSISTAYMELSSLRSEDTAMYYCARGTYYYGTRVFDYWGQG TLVTVSS (SEQ ID NO: 10) and a variable light chain of the sequence

DIVMTQSPATLSLSPGERATLSCRSSKSLQNVNGNTYLYWFQQKPGQSPQLLIYRMSN LNSGVPDRFSGSGSGTEFTLTISSLEPEDFAVYYCMQHLEYPITFGAGTKLEIK (SEQ ID NO: 11).

**5.** The combination for use according to any one of the preceding claims, wherein the antibody comprises a heavy chain constant domain of the sequence

ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTC PPCPAPELLGGPDVFLFPPKPKDTLMISRTPEVTCVVVDVSHEDPEVQFNWYVDGVEV HNAKTKPREEQFNSTFRVVSVLTVVHQDWLNGKEYKCKVSNKALPAPEEKTISKTKGQ PREPQVYTLPPSREEMTKNQVSLTCLVKGFYPSDIAVEWESNGQPENNYKTTPPMLD SDGSFFLYSKLTVDKSRWQQGNVFSCSVMHEALHNHYTQKSLSLSPGK (SEQ ID NO: 12).

**6.** The combination for use according to any one of the preceding claims, wherein the antibody comprises a light chain constant domain of the sequence

RTVAAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC (SEQ ID NO: 13).

**7.** The combination for use according to any one of the preceding claims, wherein the treatment is of non-Hodgkin's lymphoma, optionally wherein the non-Hodgkin's lymphoma is selected from the group consisting of follicular lymphoma, small lymphocytic lymphoma, mucosa-associated lymphoid tissue lymphoma, marginal zone lymphoma, diffuse large B cell lymphoma, Burkitt's lymphoma, and mantle cell lymphoma.

**8.** The combination for use according to any one of claims 1-6, wherein the treatment is of chronic lymphocytic leukemia.

**9.** The combination for use according to any one of claims 1-6, wherein the treatment is of acute lymphoblastic leukemia.

**10.** The combination for use according to any one of the preceding claims, wherein the antibody specific for CD19 and the purine analog are:

(a) formulated together in one pharmaceutical composition; or
(b) formulated in different pharmaceutical compositions.

**11.** The combination for use according to any one of claims 1 to 10, wherein the antibody specific for CD19 and the purine analog are administered simultaneously.

**12.** The combination for use according to any one of claims 1 to 9 or claim 10(b), wherein the antibody specific for CD19 and the purine analog are administered separately.

## Figure 1

**Cytotoxicity of MOR208 and FLU alone and in combination**

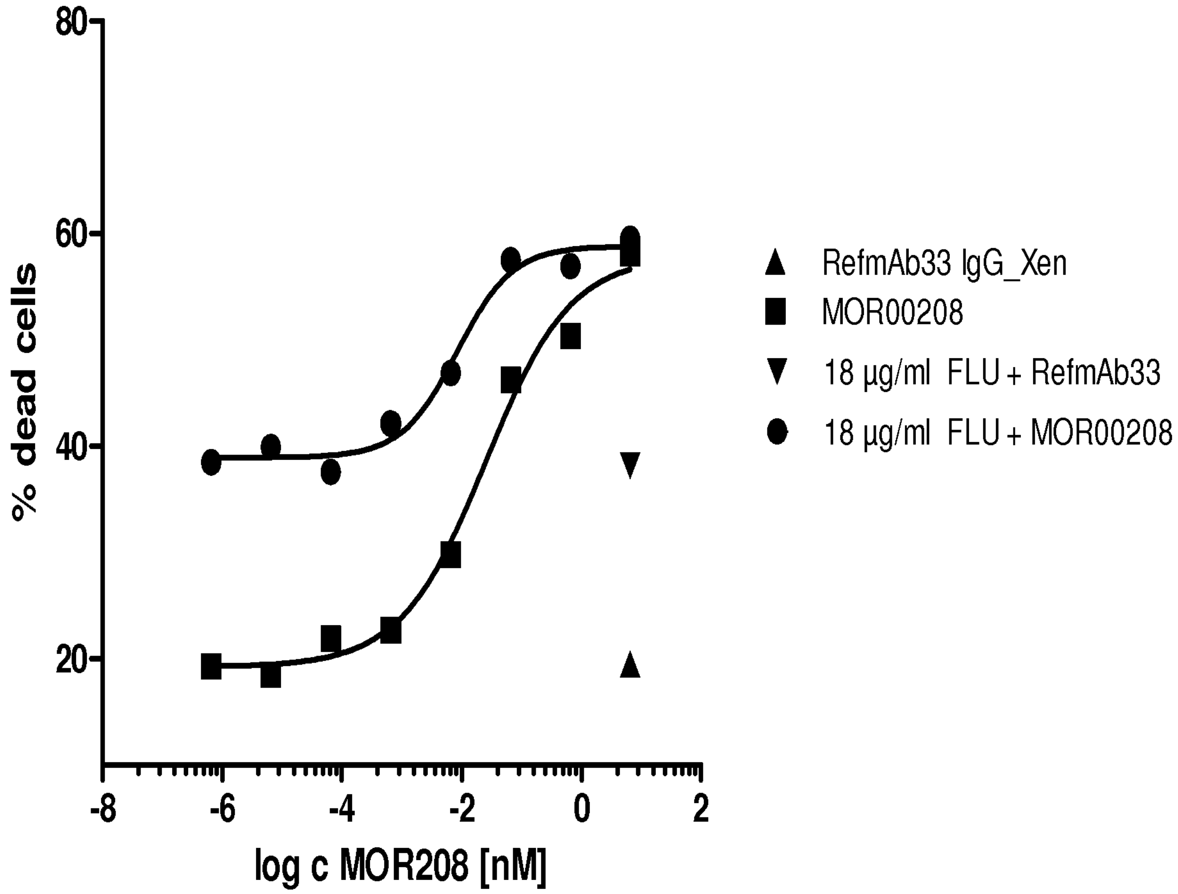

# Figure 2

## ADCC Dose response curves

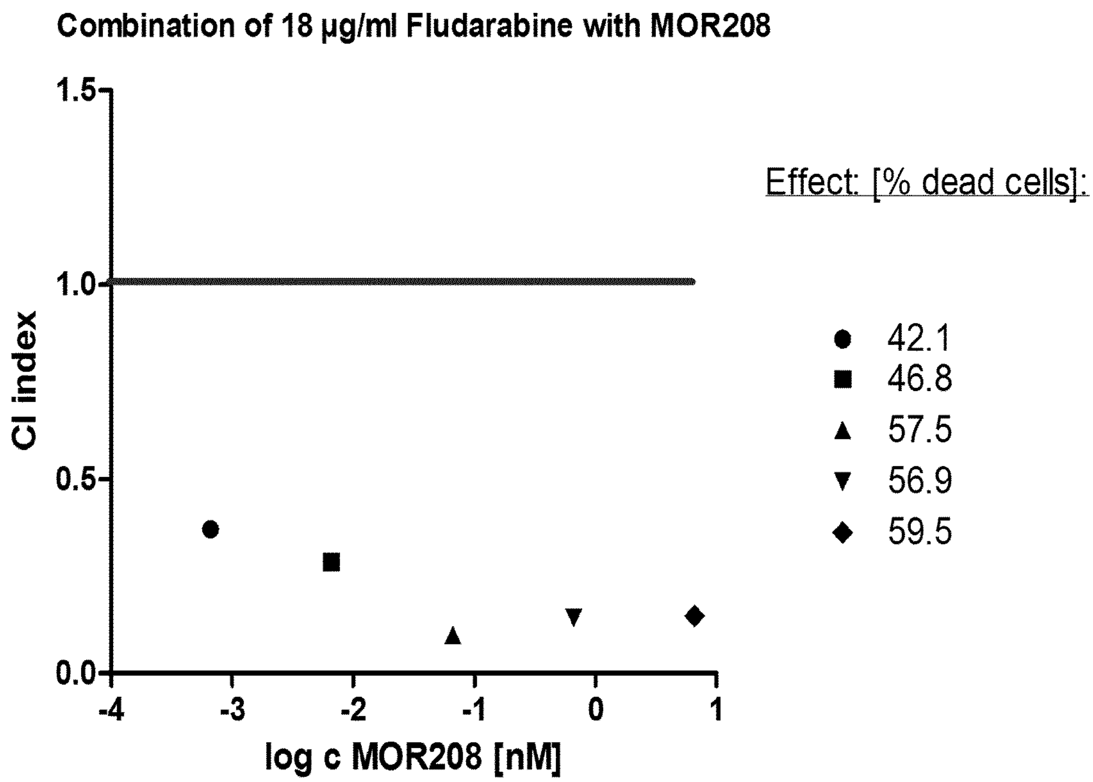

# Figure 3

The amino acid sequence of the MOR208 Variable Heavy Domain is:
(The CDRs are bolded and underlined)

EVQLVESGGGLVKPGGSLKLSCAASGYTFT**SYVMH**WVRQAPGKGLEWIGYI**NPY NDG**TKYNEKFQGRVTISSDKSISTAYMELSSLRSEDTAMYYCAR**GTYYYGTRVFDY**WG QGTLVTVSS (SEQ ID NO: 10)

The amino acid sequence of the MOR208 Variable Light Domain is:
(The CDRs are bolded and underlined)

DIVMTQSPATLSLSPGERATLSC**RSSKSLQNVNGNTYLY**WFQQKPGQSPQLLIY**R MSNLNS**GVPDRFSGSGSGTEFTLTISSLEPEDFAVYYC**MQHLEYPIT**FGAGTKLEIK (SEQ ID NO: 11)

The amino acid sequence of the MOR208 HCDR1 is: SYVMH (SEQ ID NO: 1)

The amino acid sequence of the MOR208 HCDR2 is: NPYNDG (SEQ ID NO: 2)

The amino acid sequence of the MOR208 HCDR3 is: GTYYYGTRVFDY (SEQ ID NO: 3)

The amino acid sequence of the MOR208 LCDR1 is: RSSKSLQNVNGNTYLY (SEQ ID NO: 4)

The amino acid sequence of the MOR208 LCDR2 is: RMSNLNS (SEQ ID NO: 5)

The amino acid sequence of the MOR208 LCDR3 is: MQHLEYPIT (SEQ ID NO: 6)

## Figure 4

## Sequence of Fc regions

The amino acids sequence of the MOR208 heavy chain Fc region is:

ASTKGPSVFPLAPSSKSTSGGTAALGCLVKD YFPEPVTVSWNSGALTSGVH
TFPAVLQSSGLYSLSSWTVPSSSLGTQTYICNVNHKPSNTKVDKKVEPKSCDKTHTCPPC
PAPELLGGPDVFLFPPKPKDTLMISRTPEVTCVVVDVSHEDPEVQFNWYVDGVEVHNAKT
KPREEQFNSTFRVVSVLTVVHQDWLNGKEYKCKVSNKALPAPEEKTISKTKGQPREPQV
YTLPPSREEMTKNQVSLTCLVKGFYPSDIAVEWESNGQPENNYKTTPPMLDSDGSFFLYS
KLTVDKSRWQQGNVFSCSVMHEALHNHYTQKSLSLSPGK (SEQ ID NO: 12)

The amino acids sequence of the MOR208 light chain Fc region is:

RTVAAPSVFIFPPSDEQLKSGTASVVCLLNNFYPREAKVQWKVDNALQSGNSQESVTEQ
DSKD STYSLSSTLTLSKADYEKHKVYACEVTHQGLSSPVTKSFNRGEC (SEQ ID NO: 13)

# Figure 5

Normalized specific killing; SD; MEC-1 target cells pretreated with Fludarabine (Flu) for 72 h before ADCC; pool of 3 independent experiments with 3 different effector cell donors

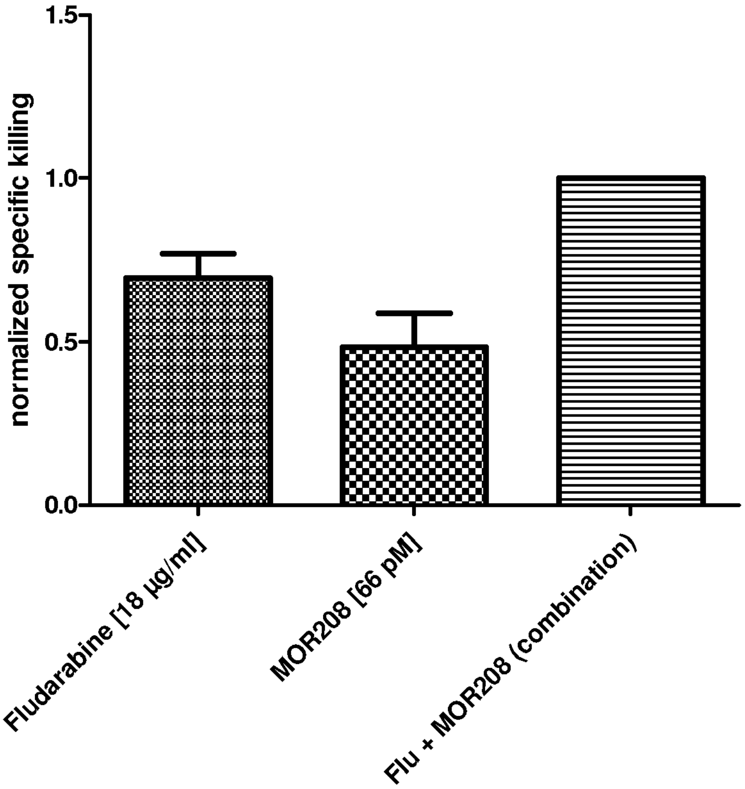


The figure shows the averages from the data shown in Table 2.

# Figure 6

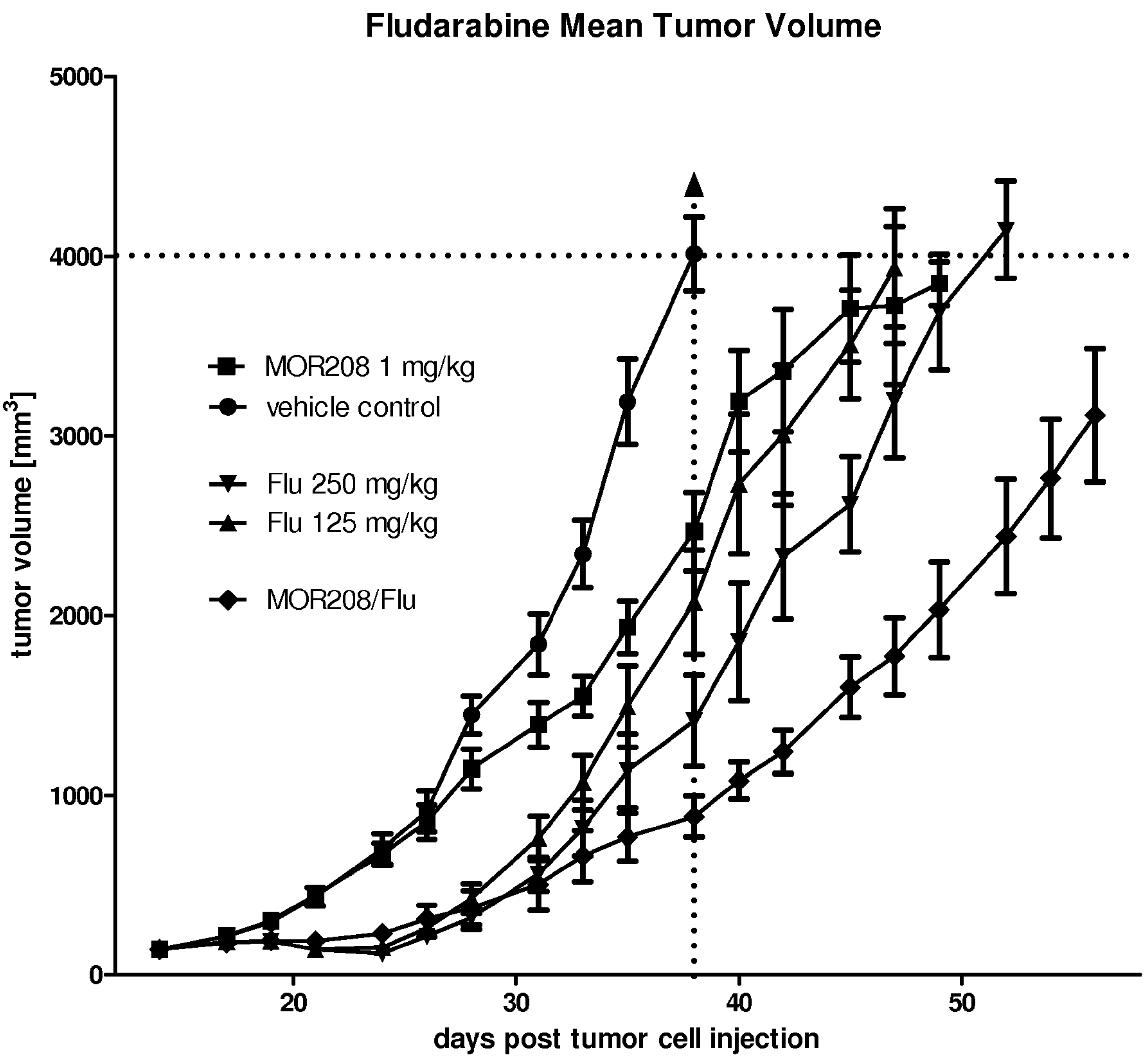

Fludarabine Mean Tumor Volume
MOR208 1 mg/kg
vehicle control
Flu 250 mg/kg
Flu 125 mg/kg
MOR208/Flu
tumor volume [mm3]
days post tumor cell injection
5000
4000
3000
2000
1000
0
20
30
40
50

**Figure 7**

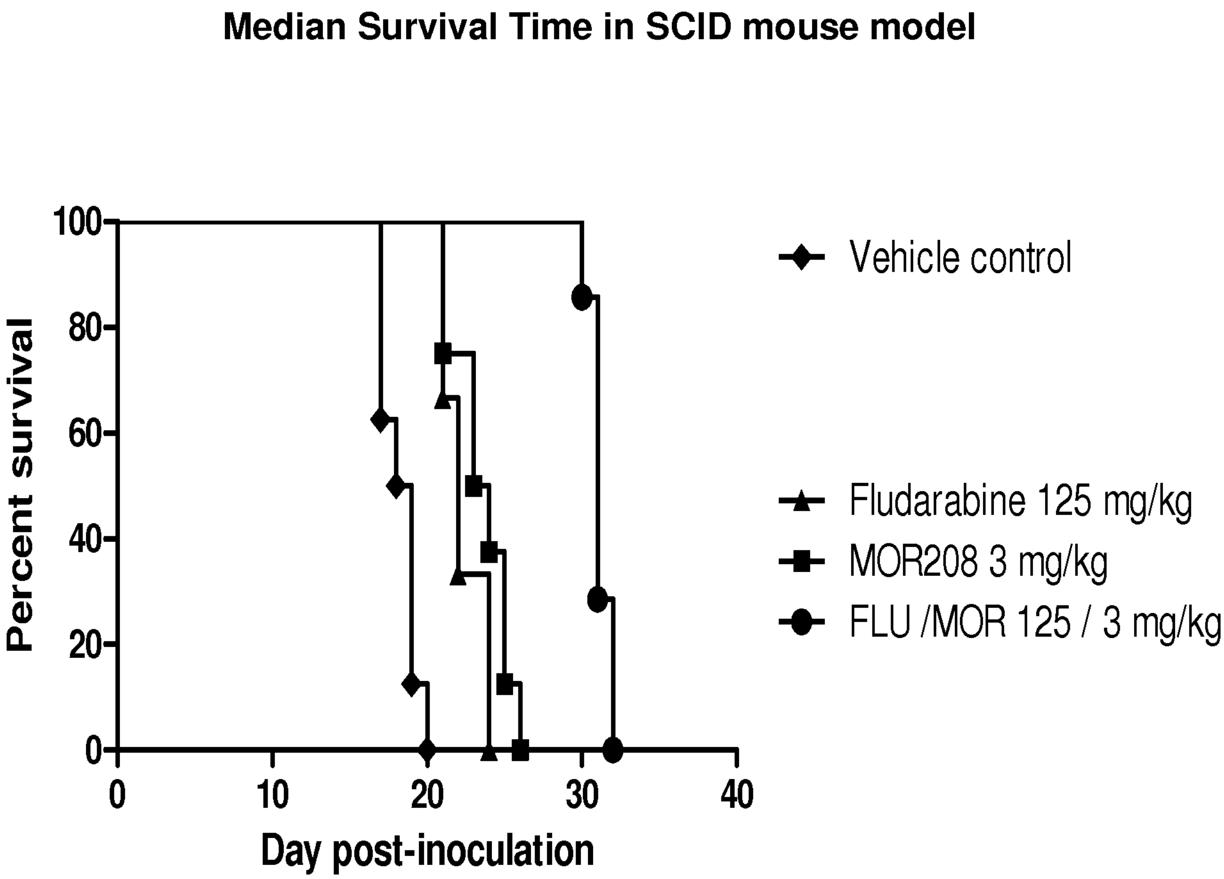
Median Survival Time in SCID mouse model
Percent survival
100
80
60
40
20
0
0
10
20
30
40
Day post-inoculation
Vehicle control
Fludarabine 125 mg/kg
MOR208 3 mg/kg
FLU /MOR 125 / 3 mg/kg

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61523862
- WO 2000074718 A
- US 2011104150 A
- WO 2007076950 A
- WO 2005012493 A
- US 7109304 B
- WO 2010053716 A
- US 12266999 B
- WO 2007002223 A
- US 8097703 B
- WO 2008022152 A
- WO 12377251 A
- WO 2008150494 A
- WO 2008031056 A
- US 11852106 B
- US 648505 B
- WO 2009052431 A
- US 12253895 B
- WO 2010095031 A
- WO 12710442 A
- WO 2010151341 A
- US 13377514 B
- US 5686072 A
- WO 2002022212 A
- US 0129026 W
- US 377251
- US 917750
- US 852106
- US 648505
- US 7968687 B
- US 710442
- WO 200348731 A
- US 4708871 A


### Non-patent literature cited in the description


- **NADLER et al.** *J. Immunol.,* 1983, vol. 131, 244-250
- **LOKEN et al.** *Blood,* 1987, vol. 70, 1316-1324
- **UCKUN et al.** *Blood,* 1988, vol. 71, 13-29
- **ANDERSON et al.** *Blood,* 1984, vol. 63, 1424-1433
- **SCHEUERMANN.** *Leuk. Lymphoma,* 1995, vol. 18, 385-397
- **GROSSBARD et al.** *Br. J. Haematol,* 1998, vol. 102, 509-15
- **TREON et al.** *Semin. Oncol,* 2003, vol. 30, 248-52
- **KOCHENDERFER et al.** Eradication of B lineage cell and regression of lymphoma in a patient treated with autologous T cells genetically engineered to recognize CD19. *Blood,* November 2010, vol. 116 (20
- The promise and potential pitfalls of chimeric antigen receptors. **SADELAIN et al.** Current Opinion in Immunology. Elsevier, 02 April 2009, vol. 21
- **ESHHAR et al.** Proceedings of the National Academy of Sciences of USA. National Academy of Science, 15 January 1993, vol. 90
- **MONTSERRAT et al.** Chronic lymphocytic leukemia treatment. *Blood Review,* 01 September 1993, vol. 7 (3
- **SCHEUERMANN et al.** CD19 Antigen in Leukemia and Lymphoma Diagnosis and Immunotherapy. *Leukemia and Lymphoma,* 1995, vol. 18, 385-397
- **TING-CHAO CHOU.** Theoretical Basis, Experimental Design, and Computerized Simulation of Synergism and Antagonism in Drug Combination Studies. *Pharmacol Rev,* 2006, vol. 58, 621-681
- **CLARKE et al.** Issues in experimental design and endpoint analysis in the study of experimental cytotoxic agents in vivo in breast cancer and other models. *Breast Cancer Research and Treatment,* 1997, vol. 46, 255-278
- **WEBB, J. L.** Enzyme and Metabolic Inhibitors. Academic Press, 1963
- **CHOTHIA C ; LESK AM.** Canonical structures for the hypervariable regions of immunoglobulins. *J Mol Biol.,* 1987, vol. 196 (4), 901-17
- **KABAT E.A ; WU T.T. ; PERRY H.M. ; GOTTESMAN K.S. ; FOELLER C.** Sequences of Proteins of Immunological Interest. US Dept. of Health and Human Services, 1991
- Epitope Mapping Protocols. Methods in Molecular Biology. Humana Press, vol. 66
- **GEYSEN et al.** *Proc. Natl. Acad. Sci. USA,* 1984, vol. 8, 3998-4002
- **GEYSEN et al.** *Proc. Natl. Acad. Sci. USA,* 1985, vol. 82, 78-182

- **GEYSEN et al.** *Mol. Immunol.,* 1986, vol. 23, 709-715
- **HOPP et al.** *Proc. Natl. Acad. Sci USA,* 1981, vol. 78, 3824-3828
- **KYTE et al.** *J.Mol. Biol.,* 1982, vol. 157, 105-132
- **TALALAY P.** Quantitative analysis of dose-effect relationships: the combined effects of multiple drugs or enzyme inhibitors. *Adv Enzyme Regul,* 1984, vol. 22, 27-55
- **JAMES et al.** Antibody-mediated B-cell depletion before adoptive immunotherapy with T cells expressing CD20-specific chimeric T-cell receptors facilitates eradication of leukemia in immunocompetent mice. *Blood,* 30 October 2009, vol. 114 (27), 5454-63
- *Breast Cancer Research and Treatment,* 1997, vol. 46, 255-278